# EUROPEAN PATENT SPECIFICATION

(11) **EP 0 852 463 B1**
(45) Date of publication and mention of the grant of the patent: **03.09.2008**
(21) Application number: 96918037.1
(22) Date of filing: 03.06.1996
(51) Int. Cl.: A01N 63/00, A01N 65/00, A61K 35/34, A61K 35/26, A61K 35/28, C12N 5/00

(54) **MYOGENIC DIFFERENTIATION OF HUMAN MESENCHYMAL STEM CELLS**
MYOGENE DIFFERENZIERUNG MENSCHLICHER MESENCHYMALER STAMMZELLEN
DIFFERENCIATION MYOGENIQUE DE CELLULES SOUCHES MESENCHYMATEUSES HUMAINES

(30) Priority: 06.06.1995 US 467223
(43) Date of publication of application: 15.07.1998
(73) Proprietor: CASE WESTERN RESERVE UNIVERSITY, Cleveland, OH 44106-4984 (US); Osiris Therapeutics, Inc., Baltimore, MD 21231-2001 (US)
(72) Inventor: CAPLAN, Arnold, I., Cleveland Heights, OH 44121 (US); YOUNG, Randell, G., Cleveland Heights, OH 44106 (US); DENNIS, James, E., Cleveland Heights, OH 44118 (US)
(74) Representative: Schnappauf, Georg
(86) International application number: PCT/US1996/008722
(87) International publication number: WO 1996/039035

(56) References cited:
- US-A- 5 435 999
- US-A- 5 486 359
- BRUDER SCOTT P ET AL: "Mesenchymal stem cells in bone development, bone repair, and skeletal regeneration therapy" JOURNAL OF CELLULAR BIOCHEMISTRY, vol. 56, no. 3, 1994, pages 283-294, XP002082633 ISSN: 0730-2312
- DARMON M ET AL: "5-Azacytidine is able to induce the conversion of teratocarcinoma-derived mesenchymal cells into epithelia cells." THE EMBO JOURNAL. MAY 1984, vol. 3, no. 5, May 1984 (1984-05), pages 961-967, XP001024628 ISSN: 0261-4189
- DATABASE BIOSIS [Online] BIOSCIENCES INFORMATION SERVICE, PHILADELPHIA, PA, US; 1994, AURADE FREDERIC ET AL: "Myf5, MyoD, myogenin and MRF4 myogenic derivatives of the embryonic mesenchymal cell line C3H10T1/2 exhibit the same adult muscle phenotype" XP002286561 Database accession no. PREV199497220486 & DIFFERENTIATION, vol. 55, no. 3, 1994, pages 185-192, ISSN: 0301-4681
- ACTA ANAT., January 1989, Vol. 134, No. 1, MORI N. et al., "Ultrastructural Findings in the Wound Healing of the Colonic Mucosa of Rabbits", pages 82-88.
- CLINICS IN PLASTIC SURGERY, July 1994, Vol. 21, No. 3, CAPLAN A.I., "The Mesengenic Process", pages 429-435.
- MUSCLE & NERVE, December 1995, Vol. 18, No. 12, WAKITANI S. et al., "Myogenic Cells Derived from Rat Bone Marrow Mesenchymal Stem Cells Exposed to 5-Azacytidine", pages 1417-1426.

## Description

This invention relates to the field of muscle regeneration and, more particularly, to the production of dystrophin-positive cells in dystrophin-negative subjects.

A mesenchymal stem cell (MSC) is a pluripotential progenitor cell that has the capacity to divide many times and whose progeny eventually gives rise to mesodermal tissue such as: cartilage, bone, muscle, fat and tendon. A population of these pluripotential stem cells has been shown to be present in embryonic limb buds of chicken, mouse and human (2, 5, 13, 26, 27). Such stem cells have also been shown to exist in postnatal and adult organisms. Friedenstein, Owen (8, 22) and others reported that cells derived from bone marrow have the capability to differentiate into osteogenic cells when assayed in diffusion chambers. It has been documented that such cells when loaded in ceramic cubes and implanted to subcutaneous or intramuscular sites, possess the ability to form bone and cartilage tissue (10, 21). Furthermore, the periosteum has been reported to contain MSCs with the same ability to form bone or cartilage in ceramic cubes or diffusion chambers (20).

With regard to myogenic potential of MSCs, rat and mouse clonal embryonic cell lines have been shown to transform into myoblasts and form myotubes after exposure to 5-azacytidine or 5-azadeoxy cytidine. This work is disclosed in copending, commonly assigned U.S. application US 1995/377461, filed January 24, 1995 and entitled "Lineage-Directed Induction of Human Mesenchymal Stem Cell Differentiation." The same cells also exhibit adipocytic and chondrogenic phenotypes (11, 17, 21, 37). However, the expression of myogenic properties of stem cells harvested from postnatal sources has not been observed in humans.

The mdx strain of mice is well recognized and has been utilized as an animal model correlated with Duchenne-type human muscular dystrophy (5). Although mdx mice do not exhibit severe clinical features of myopathy, their skeletal and cardiac muscles are composed of predominantly dystrophin-negative myofibers and show an extensive myopathic lesion accompanied with muscle fiber necrosis and degeneration (10). Injection of myoblasts (satellite cell-derived), also referred to herein as muscle precursor cells(MPCs), has been observed to result in the conversion of dystrophin-negative myofibers to dystrophin-positive ones (25, 30), giving hope that this procedure may provide a useful treatment for the patients affected with inherited myopathies (18, 24, 26). One major drawback in this myoblast transfer therapy is that a large number of myoblasts are needed to bring about a clinically relevant effect.

The present invention is based on the discovery by the inventors that suitable environmental conditions induce human mesenchymal stem cells (hMSCs) to express a myogenic phenotype. Such conditions can be established in developing myogenic cell culture or in regenerating and reforming dystrophin-positive muscles. The procedures described here elucidate the behavior of marrow- or periosteal- derived hMSCs in the presence of muscle precursor cells (MPCs) *in vitro* and *in vivo*.

Because, *inter alia*, hMSCs can be mitotically expanded in culture and their harvest is less destructive than muscle biopsies used to harvest myoblasts, these cells have been discovered by the inventors to have significant advantages over the use of myoblasts in treating myopathies. Additionally, there are several sources of hMSCs in the human body, and their harvest is less destructive than muscle biopsies used to harvest MPCs.

With the above in mind, the inventors studied the behavior of hMSCs co-cultured with myoblasts, and also injected syngeneic normal MSCs into muscles of the mdx mouse to evaluate their capability to produce normal muscle fibers.

Accordingly, in one aspect the invention provides a composition of matter which comprises a mixture of isolated muscle precursor cells and isolated human mesenchymal stem cells. Preferably, the mesenchymal stem cells are marrow-derived or periosteum-derived. Also, the composition is preferably a composition of entirely human cells. The composition can further include a myoinductive agent, such as 5-azacytidine or 5-azadeoxycytidine.

Another aspect of the invention provides a composition of matter which comprises a mixture of isolated muscle precursor cells and isolated periosteum cells. Here also, the composition is preferably a composition of entirely human cells. This composition also can further include a myoinductive agent, such as 5-azacytidine or 5-azadeoxycytidine.

Another aspect of the invention provides a method for inducing isolated human mesenchymal stem cells to differentiate into myogenic cells by maintaining the cells in the presence of muscle precursor cells, wherein the isolated human mesenchymal stem cells are maintained in the presence of muscle precursor cells *in vitro*.

Another aspect of the invention provides use of a myogenic cell producing amount of isolated human mesenchymal stem cells or of any of the compositions of the invention for the manufacture of a medicament for producing dystrophin-positive myogenic cells in an individual by administering it to the individual.

Another aspect of the invention provides the use of a muscle regenerative amount of isolated human mesenchymal stem cells or of any of the compositions of the invention for the manufacture of a medicament for effecting muscle regeneration in an individual in need thereof by administering to an individual in need of muscle regeneration.

Another aspect of the invention provides the use of an amount of isolated human mesenchymal stem cells or of any of the compositions of the invention effective to produce dystrophin-positive myogenic cells in an individual for the manufacture of a medicament for treating muscular dystrophy in an individual so afflicted by administering to the individual.

The following is a brief description of the drawings which are presented only for the purposes of further illustrating the invention and not for the purposes of limiting the same.

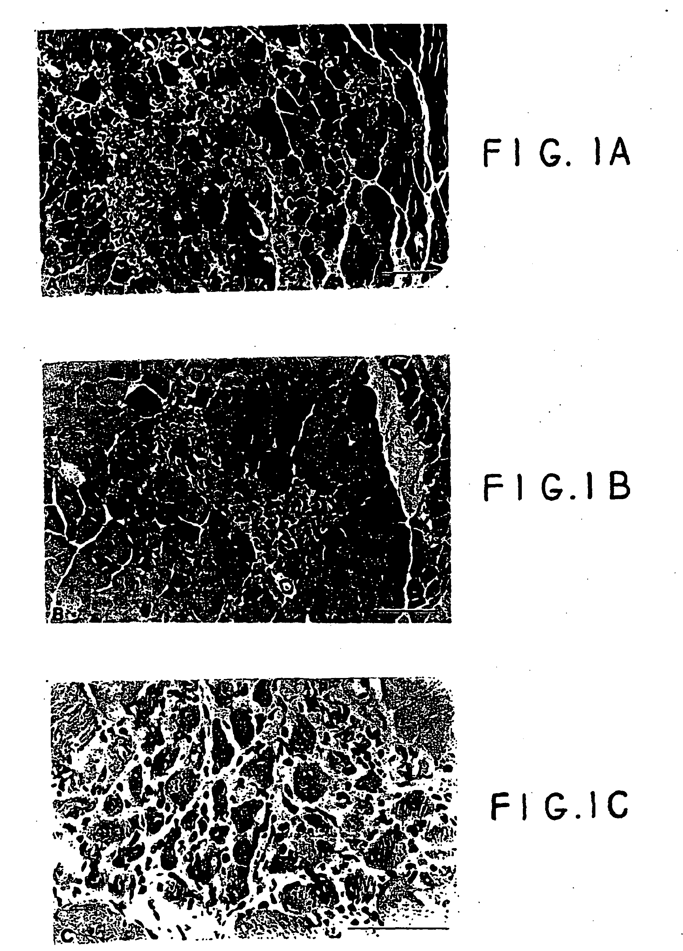

Figures 1A-1C are photomicrographs showing the histology of the right tibialis anterior muscle of mdx mouse at 6 weeks after injection (A). There is a large area of a typical myopathic lesion accompanied with cellular infiltration. Inside the lesion, swollen and vacuolated myotubes are found. At 8 weeks post-injection (B), the regenerating area contains clustered muscle fibers of small diameter, surrounded by large closely packed normal muscle fibers. At a higher magnification (C), a typical regenerating fibers are shown having large centrally located nuclei. Bar = 100 µm.

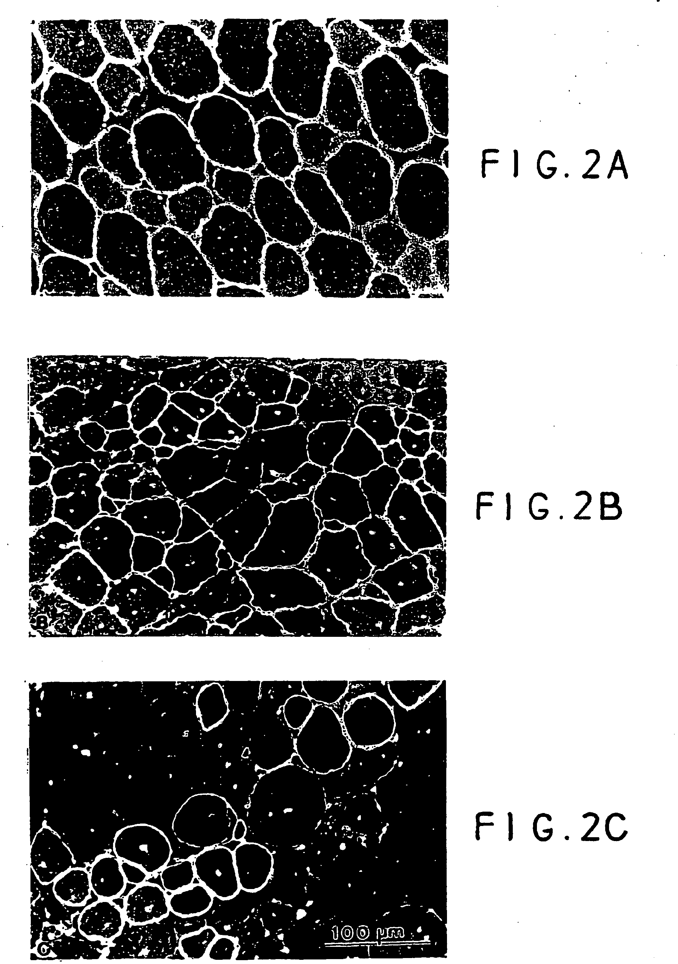

Figures 2A-2C are photomicrographs showing dystrophin expression in the muscle of normal mouse (A). The entire circumference of muscle fibers are clearly stained with anti-dystrophin antibody. Anti-dystrophin antibody localization in the muscle of mdx mouse 8 weeks after injection of myoblasts (B). Immunostaining clearly demonstrates multiple dystrophin-positive fibers surrounded by dystrophin-negative myotubes. The shape and size of the positive fibers are more varied than those of a normal muscle. There are few dystrophin-negative fibers intermingled with the positive fibers (*). Dystrophin expression in the muscle of an *mdx* mouse 10 weeks post-injection with MSCs (C). The intensity of staining is almost same as that in the muscle injected with myoblasts. There are contiguous dystrophin-positive muscle fibers, foaming a cluster.

Skeletal muscles develop in a specific sequence of cellular differentiation, which includes: the commitment of progenitor cells into myoblasts, the proliferation of myoblasts, their fusion to form multinucleated myotubes and the sequential expression of muscle specific proteins. Each of these steps is controlled by a complex spectrum of intrinsic and extrinsic biological factors (38). Particularly, in the process of myoblast fusion, there is a clear molecular recognition mechanism which allows myoblasts to fuse only to cells of their own lineage, prohibiting their fusion to other cell types such as osteogenic, chondrogenic and fibroblastic cells (38,40).

Mesenchymal stem cells are the formative pluripotential blast cells found *inter alia* in bone marrow, blood, dermis and periosteum that are capable of differentiating into any of the specific types of mesenchymal or connective tissues (*i.e*. the tissues of the body that support the specialized elements; particularly adipose, osseous, cartilaginous, elastic, and fibrous connective tissues) depending upon various influences from bioactive factors, such as cytokines. Although these cells are normally present at very low frequencies in bone marrow, a process for isolating, purifying, and greatly replicating these cells in culture, *i.e*. *in vitro*, is disclosed in PCT Published Application No. WO 92/22584 (published 23 December 1992).

Homogeneous human mesenchymal stem cell compositions are provided which serve as the progenitors for all mesenchymal cell lineages. MSCs are identified by specific cell surface markers which are identified with unique monoclonal antibodies. The homogeneous MSC compositions are obtained by positive selection of adherent marrow or periosteal cells which are free of markers associated with either hematopoietic cell or differentiated mesenchymal cells. These isolated mesenchymal cell populations display epitopic characteristics associated with only mesenchymal stem cells, have the ability to regenerate in culture without differentiating, and have the ability to differentiate into specific mesenchymal lineages when either induced in vitro or placed *in vivo* at the site of damaged tissue.

In order to obtain human mesenchymal stem cells, it is necessary to isolate rare pluripotent mesenchymal stem cells from other cells in the bone marrow or other hMSC source. Bone marrow cells may be obtained from iliac crest, femora, tibiae, spine, rib or other medullary spaces. Other sources of human mesenchymal stem cells include placenta, umbilical cord, fetal and adolescent skin, and blood.

The method of their isolation comprises the steps of providing a tissue specimen containing mesenchymal stem cells, adding cells from the tissue specimen to a medium which contains factors that stimulate mesenchymal stem cell growth without differentiation and allows, when cultured, for the selective adherence of only the mesenchymal stem cells to a substrate surface, culturing the specimen-medium mixture, and removing the non-adherent matter from the substrate surface.

Compositions having greater than 95%, usually greater than 98% of human mesenchymal stem cells can be achieved using the previously described technique for isolation, purification and culture expansion of MSCs. The desired cells in such compositions are identified as SH2⁺, SH3⁺, SH4⁺ and CD⁻ and are able to provide for both self renewal and differentiation into the various mesenchymal lineages. Ultimately, repair and regeneration of various mesenchymal tissue defects could be accomplished starting from a single mesenchymal stem cell.

### Example 1

### Dystrophin Expression in mdx Mice Using hMSCs

Mdx mice have been utilized as an animal model of Duchenne-type human muscular dystrophy. Although *mdx* mice do not exhibit severe clinical features of myopathy, their skeletal and cardiac muscles are composed of predominantly dystrophin-negative myofibers and show an extensive myopathic lesion accompanied with muscle fiber necrosis and degeneration. Injection of myoblasts (satellite cell-derived) has been observed to result in the conversion of dystrophin-negative myofibers to dystrophin-positive ones, which gives hope that this procedure may provide a useful treatment for patients affected with inherited myopathies. Therefore, the *mdx* mouse was adopted as an experimental animal model in these studies. One major drawback in this myoblast transfer therapy is that a large number of myoblasts are needed to bring about a clinically relevant effect. Because MSCs can be mitotically expanded in culture and their harvest is less destructive than muscle biopsies used to harvest myoblasts, these cells may be an alternative source of cells for treating myopathies. With the above in mind, we have studied the behavior of MSCs co-cultured with myoblasts or injected into either syngeneic normal muscle or into the *tibialis anterior* muscle of mdx mice, in order to evaluate the capability of MSCs to produce normal muscle fibers.

### MATERIALS AND METHODS

### Bone Marrow Cell Preparation

As sources of bone marrow MSCs, femora of male mice (C57B1/10 SNJ, Jackson Laboratories, Bar Harbor, ME) of 6 to 10 weeks of age were used. After the soft tissue was completely removed to avoid contamination by myogenic precursors, the distal and proximal ends of the femora were cut with a rongeur and the marrow plugs were removed from the shafts of the bones by expulsion with a syringe filled with complete medium and fitted with, for rats, an 18-gauge needle and, for mice, a 20-gauge needle. Complete medium consisted of BGJ_{b} (Fitton-Jackson Modification; Sigma; St. Louis, MO) with antibiotics (100 µ/ml sodium penicillin G, 100 µg/ml streptomycin sulfate and 0.25 µg/ml amphotericin B: Gibco BRL, Grand Island, NY), and 10% fetal calf serum (selected batches; JRH Biosciences, Lenexa, KS). Marrow cells were dispersed several times through 20- and 22-gauge needles. The cells were gently centrifuged, resuspended in complete medium, counted and plated at a density of 5.0 x 10⁷ cells/100-mm dish. At 3 days of culture, the medium was removed, the dishes were washed twice with Tyrode's salt solution (Sigma, St. Louis, MO) and fresh complete medium added; the medium was changed twice a week.

### Preparation of Mouse Myoblasts

Muscle tissue was obtained from the *tibialis anterior* and *quadraceps* muscles of three-week-old mice. The dissected muscle was digested with 0.2% trypsin for 35 min and the reaction terminated by the addition of fetal bovine serum at one half the sample volume. The cell suspension was centrifuged for 5 min at 300 xg, resuspended in 10 ml of DMEM-LG containing 20% fetal calf serum, triturated at least 20 times with a 10-ml disposable pipette, and then passed through a 100-µm Nitex filter (to remove cell aggregates and debris). Prior to seeding, the cells were preplated into a 100-mm culture dish and incubated for 30 min to reduce the number of fibroblasts. The nonadherent muscle precursor cells were counted in a hemocytometer and the cell density was adjusted to 1.0 x 10⁵ cells/ml with DMEM-LG supplemented with 20% fetal calf serum. Cells were seeded onto 35 mm plates at 1.0 x 10⁵ cells per plate, in 1 ml, and the medium changed every 2 days. At the onset of cell fusion (4-5 days), the medium was changed to DMEM-LG with 5% fetal calf serum. The cultures were maintained until the overt formation of myotubes has ceased; usually by 11 days following seeding.

### In Vivo Injections

Breeding pairs of both mdx mice (C57b1/10mdx) and normal mice (C57b1/10snj) were purchased from Jackson Laboratories. Newborn *mdx* mice were raised for 3 weeks, separated from their parents and immediately used as injection hosts. Normal newborn mice were sacrificed within 3 days of birth for collection of syngeneic myoblasts. Normal adult male mice were utilized as a source of bone marrow-derived MSCs.

Myoblasts were obtained from normal syngeneic newborn muscle tissue as described above, then centrifuged, washed with Tyrode's salt solution and counted. MSCs were released with trypsia-EDTA after 10 to 14 days of primary culture, washed with Tyrode's salt solution and counted. The densities of both cell suspensions were adjusted with serum-free BGJ_{b} to 5.0 x 10⁵ cells/5 µl.

*Mdx* mice were anesthetized by limited exposure to CO₂ gas. The hindlimbs were sterilized with 70% ethanol, and a longitudinal skin incision was made along the anterior edge of the tibia with a No. 11 scalpel. The *tibialis anterior* muscle was identified and a needle connected to a microliter syringe (Hamilton Company, Reno, NV) containing 5 µl of cells was inserted into the interior of the muscle at a low angle, and the full 5 µl of cells were injected into the middle of the muscle. The right *tibialis anterior* was injected with cells and the left side injected with BGJ_{b} medium only. The skin was sutured in an ordinary manner and closed.

### Tissue Section Immunohistochemistry

At 6, 8 and 10 weeks after injection, mice were sacrificed, and the muscle mass located on the anterior surface of the tibia was excised. The mass of each muscle sample was measured prior to cutting the muscle in half. The proximal half was immersed in OCT compound (Tissue-Tek, Miles Laboratories, Inc., Napperville, IL) and then snap-frozen in liquid nitrogen-cooled in 2-methylbutane for cryosectioning and immunohistochemical staining; the distal half was fixed with 10% neutral formalin for routine histology.

The frozen samples were sectioned with a cryostat, and 8 µm sections were placed on glass slides coated with poly-L-lysine. Prior to staining, sections were blocked in phosphate-buffered saline containing 20% horse serum (JRH Biosciences, Lenexa, KS; PBS+HS, pH 7.4). The sections were immunostained with anti-dystrophin antibody as described above. Following incubation in the first and second antibodies, they were washed and incubated for 15 min with Texas red conjugated avidin (Organon Teknika Corp., West Chester, PA) diluted 1 to 6000 with PBS+HS and washed 4 times in PBS+HS for 5 min. The slides were mounted in glycerol: PBS (9:1), pH 8.5 containing 0.01 M p-phenylenediamine (PPD) (Eastman Kodak, Rochester, NY) and observed with a fluorescent microscope (BH-2, Olympus, Tokyo).

### Histologic Evaluation

For analysis of the dystrophin content in the transverse section of the muscle, the section was subdivided into 6 areas and a micrograph of the region containing the largest number of dystrophin-positive myofibers in each area was recorded with Kodak TMAX 400 film. From full frame prints, the number of dystrophin-positive and -negative myofibers was tabulated and the percentage of positive to total number of myofibers in the 6 areas was calculated. The average values of the right muscle (cell-injected) were compared with that of the left (cell medium-injected). Sections of the other half of the muscle samples were stained with hematoxylin and eosin for histologic evaluation.

### RESULTS

### In vivo Muscle injections

A total of 52 mdx mice were used for the injections in this study. Muscles were harvested at 6, 8 and 10 weeks after injection; 29 mice were injected with MSCs and 23 with myoblasts. During harvest, muscles were dissected *in toto* and immediately weighed. Table II contains the mass ratios between right and left muscles harvested at 6, 8 and 10 weeks after injection. The data were then analyzed both in total and according to sex; this data is summarized in Table 1.

**Table 1**

| **Summary of 6, 8 and 10 Week Post-Injection Muscle Mass** | | | |
|---|---|---|---|
| **mg muscle (standard deviation)** | | | |
| | **Total Female** | **Total Male** | **Total F + M** |
| ***Myoblast-injected*** | | | |
| Cell-injected | 93.2 (8.5)^{†} | 100.6 (10.1)^{§} | 97.5 (10.0)^{¶} |
| Medium-injected | 85.7 (12.6) | 88.9 (26.0) | 87.6 (21.1) |
| Percent increase | 8.8 | 13.1 | 13 |
| Number | 10 | 13 | 23 |

| ***Mesenchymal Stem cell-injected*** | | | |
|---|---|---|---|
| Cell-injected | 78.2 (12.2)^{¶} | 97.9 (12.2) | 85.6 (15.9)^{┌} |
| Medium-injected | 74.7 (10.5) | 96.8 (9.5) | 83.1 (14.7) |
| Percent increase | 5.4 | 0.9 | 3.0 |
| Number | 18 | 11 | 29 |

All cell-injected muscle groups showed statistically significant increases in muscle mass compared to contralateral controls except for the male MSC-injected group. There was an increase in muscle mass of this group but not statistically significant (p value = 0.7). The myoblast-injected muscle showed nearly a 4-times greater percent change in muscle mass (11.1%) than that of MSC-injected muscle (3.0%).

The histology of the muscle samples showed various degrees of degeneration and/or regeneration in both MSC-and periosteal cell-injected groups. At 6 weeks after injection, large foci of cellular infiltration were found (Fig. 1A). However, at 8 to 10 weeks, these areas were reduced in size and were surrounded by myotubes (Fig. 1B). The transverse sections appeared normal, except that the nuclei were centrally located as compared to the normal peripheral location (Fig. 1C). The frequency of small, basophilic, centrally nucleated and vacuolated myotubes increased in number with time. Although there were areas of fat cell accumulation in some few samples, calcified myofibers and massive fibrosis were not observed. There was no evidence of bacterial infection in any of the muscle specimens.

Immunohistochemical staining for dystrophin served as a marker for normal muscle differentiation. In the normal mouse (snj), dystrophin is localized to the entire circumference of the plasmalemma on every myotube (Fig. 2A). In the myoblast-injected group, a wide variation of the extent of dystrophin-positive myofibers was observed. The shape and diameter of the positive fibers were more irregular than those of normal syngeneic mice (Fig. 2B). Of interest was the finding that some of the fibers with the centrally located nuclei were also dystrophin-positive.

Quantitation of immunoreactivity for samples harvested at 6, 8, and 10 weeks after injection showed a consistent pattern for the percentage of dystrophin-positive fibers within each experimental group (Table 2).

**Table 2**

| **Incidence of Dystrophin-Positive Muscle Fibers** | | | |
|---|---|---|---|
| **Proportion of Positive Fibers (%)** | | | |
| | **6 wk** | **8 wk** | **10 wk** |
| ***Injected*** | | | |
| MSC (R) | 2.4±1.7 (9) | 2.6±1.6 (9) | 5.7±9.4 (11) |
| Sham (L) | 0.3±0.5 (9) | 0.5±0.6 (9) | 0.3±0.5 (11) |
| MPC (R) | 15.4±19.1 (9) | 35.8±16.7 (7) | 26.6±23.2 (8) |
| Sham (L) | 0.8±0.8 (8) | 0.3±0.6 (7) | 3.0±2.0 (8) |

At each harvest time, the myoblast-injected samples had, by far, the largest regions of dytrophin-positive myotubes, followed by the MSC-injected group, and then by the medium-injected control group. The percentage of positive myofibers for the myoblast-injected group was from 5 to 10 times greater than that for the MSC-injected group. However, the MSC-injected group had 8 to 19 times more dystrophin-positive myofibers than the medium-injected muscles. There was a significant difference in the average values between the MSC-injected and medium-injected muscles at 6 and 8 weeks (P<0.05, two-way ANOVA) (Table II). The intensity of staining among the test groups was indistinguishable from that of the myoblast-injected group (Fig. 2C).

At 6 weeks after injection, in the medium-injected group, four samples showed 1 to 8 positive fibers and the other 5 were completely negative. Most of the positive fibers were scattered except in one animal where a cluster containing 8 adjacent positive fibers was found. In MSC-injected muscles, clusters with more than 3 positive fibers were more frequently found. However, the number of the positive fibers in a cluster was limited to under 8. At 8 weeks, the distribution pattern of the positive fibers was similar to that observed in the 6-week samples. At 10 weeks, there were no dystrophin-positive fibers in 8 of 11 medium-injected muscles. On the other hand, in MSC-injected muscles, 7 muscles showed a cluster containing more than 6 contiguous positive fibers and in one case a marked formation of positive fibers was found.

### DISCUSSION

Skeletal muscles develop in a specific sequence of cellular differentiation, which includes: the commitment of progenitor cells into myoblasts, the proliferation of myoblasts, their fusion to form multinucleated myotubes and the sequential expression of muscle-specific proteins. Each of these steps is controlled by a complex spectrum of intrinsic and extrinsic biological factors. Particularly, in the process of myoblast fusion, there is a clear molecular recognition mechanism which allows myoblasts to fuse only to cells of their own lineage and prohibits their fusion to other cell types such as osteogenic, chondrogenic and fibroblastic cells.

In order to account for the spontaneous dystrophin-expression by somatic cell mutation in *mdx* mice with advancing age, the proportion of dystrophin-positive fibers was determined for the MSC-injected right and medium-injected left muscles. Although the incidence of positive fibers was lower in MSC-injected muscles than that of muscles injected with myoblasts, it was significantly higher than that of the controls somatic mutation at each sampling time. Several investigators have proposed that the *mdx* mouse can be used as an animal model to study muscular regenration. It has been shown that myopathic lesions similar to Duchenne-type human muscular dystrophy are manifest in young mdx mice at age 3 to 6 weeks, since no apparent sex-related difference in histology was detected. Injections of reparative cells were done at age 3 weeks into both male and female MDX mice. Our results show that the average rate of conversion was highest at 8 weeks after injection (36%). In comparison, the muscles injected with MSCs showed a cluster of dystrophin-positive myofibers at a significantly lower frequency than the myoblast-injected muscles.

The *in vivo* experiments presented here indicate that MSCs are able to fuse with normal and mdx myoblasts and, when injected, can contribute to increased muscle mass, and increased numbers of dystrophin-positive fibers as compared to controls. From these data, taken together, it is reasonable to interpret that some of the increase in muscle mass can be attributed to myoblast- or MSC-induced conversion of dystrophin-negative fibers to dystropin-positive fibers. We postulate that the host provides the signals to cause the MSCs to exhibit a myogenic potential. The result of *in vivo* injectin experiments indicate that implanted bone marrow-derived MSCs differentiate into myogenic cells in regnerating muscles of mdx mice. However, the frequency for MSC myogenic conversion is low, so we cannot rule out co-fusion as a possible mechanism for our observations. In summary, MSC's are able to fuse with myotubes and express dystrophin, and are a reservoir which can be utilized for dystrophy-related cell therapies.

We hypothesize that the differentiation efficiency may be increased by the regeneration conditions inherent in *mdx* mice. Therefore, implantation of MSCs in combination with myoblasts or inductive reagents to promote muscle regeneration result in more efficient conversion of dystrophin-negative to -positive myofibers in the muscle of mdx mice.

### Example 2

### Induction of Myogenisis in Human Volunteers

### Bone Marrow Cell Preparation

Bone marrow cells aspirated from the iliac crests of healthy adult human volunteers are used. After soft tissue is completely removed to avoid contamination of myogenic precursors, the marrow is triturated with a syringe filled with Complete Medium and fitted with an 18-gauge needle. The Complete Medium was made up of DMEM-LG (Dulbecco's Modified Eagle's Medium - Low Glucose, Gibco BRL, Grand Island, NY) as more fully described in copending U.S. Serial No. 08/420,297, filed April 11, 1995. The Medium contains antibiotics (100 µ/ml sodium penicillin G, 100 µg/ml streptomycin sulfate and 0.25 µg/ml amphotericin B: Gibco BRL, Grand Island, NY) and is supplemented with 10% fetal calf serum (selected batches). Marrow cells are dispersed several times through 18- and 20-gauge needles. The cells are gently centrifuged, resuspended in Complete Medium, counted and plated at a density of 5.0 x 10⁷ cells/100 mm-dish. At 3 days of culture, the medium is removed, the dishes are washed twice with Tyrode's salts solution (Sigma, St. Louis, MO) and fresh complete medium is added. The medium is changed twice a week.

### Periosteal-Derived Cell Preparation

Periosteal cells are obtained from the proximal tibiae of volunteers. The tibial periostea are digested with 0.3% collagenase for 2 hours in a 37°C water bath. At the end of the digestion period, a volume of fetal bovine serum equal to half the volume of the sample is added. Periosteum-derived cells (periosteal cells) are collected by centrifugation. The pellet of cells and debris are resuspended in 8 ml of Complete Medium and seeded onto 100-mm culture dishes. The plated cells are cultured at 37°C in 95% humidified air plus 5% CO₂, and the medium is changed every 3 days.

### Preparation of Myoblasts

Muscle tissue is obtained from muscle biopsies. The dissected muscle is digested with 0.2% trypsin for 35 min. and the reaction is terminated by adding fetal bovine serum at one half the sample volume. The cell suspension is centrifuged for 5 min. at 300 xg, resuspended in 10 ml of DMEM-LG containing 20% fetal calf serum, triturated at least 20 times with a 10 ml disposable pipette and then passed through 110 µm Nitex filter (to remove cell aggregates and debris). Before seeding, the cells are preplated in a 100-mm culture dish and incubated for 30 min. to reduce the number of fibroblasts. The nonadherent muscle precursor cells are counted in a hemocytometer and the cell density is adjusted to 1.0 x 10⁵ cells/ml with DMEM-LG supplemented with 20% fetal calf serum. Cells are seeded onto 35 mm plates at 1.0 x 10⁵ cells per plate, in 1 ml, and the medium is changed every 2 days. At the onset of cell fusion (4-5 days), the medium is changed to DMBM-LG with 5% fetal calf serum. The dishes are observed until myotubes form, usually 11 days following initial seeding.

### Alternate Preparation of Myoblasts

Myoblasts are also obtained as above. The bone and cartilage are cleared with fine forceps under a dissecting microscope. The dissected muscle tissue is transferred to a sterile tube, 2 ml of Eagle's medium with Barle's salts is added and the tissue is vortexed for 30-60 seconds. Subsequently, 8-9 ml of Complete Medium [Eagle's Minimum Essential Medium with Earle's salts containing 10% horse serum, 5% embryo extract and antibiotics] are added. The cell suspension is passed through 2 thicknesses of sterile cheesecloth two times and the cells are counted in a hemocytometer. The cell density is adjusted with Complete Medium to 2.0 x 10⁵/ml and one ml is aliquoted onto each 35 mm dish.

### Method of Co-culture

A 1 ml of suspension of MPCs is dispersed on gelatin-coated 35-mm culture dishes. Periosteal-derived cells (PCs) labeled with [³H] -thymidine are trypsinized for 5 min. at 37°C with 0.25% trypsin/1 mM ethylenediamine tetra-acetic acid (Gibco BRL, Grand Island, NY), the reaction is terminated by the addition of half volume of calf serum and cells are collected by centrifugation. Subsequently, three different cell suspensions are made at cell densities of 1.0 x 10⁵, 2.0 x 10⁵ and 3.0 x 10⁵ cells/ml separately with Complete Eagle's and Earl's salts Medium. Then, 1 ml of each cell suspension is added to the dishes in which MPCs are seeded previously and mixed with each other. Cells are cultured at 37°C in CO₂ incubator. The medium is changed twice a week.

### Activity of Creatine Kinase

Every 2 days after the initiation of the co-culture until day 11, cells are harvested from the dishes by washing twice with cold Tyrode's salt solution twice and stored at -70°C until needed. After thawing, 1 ml of 0.05 M glycylglycine (pH 6.75, Sigma, St. Louis, MO) is added into each dish, and cells are scraped off with a polypropylene policeman, suspended, sonicated for 30 sec. in a Sonifier Cell Disrupter (Model W140D, Branson Sonic Power Co., Plainview, NY) and centrifuged at 12,000 xg for 5 min. The activity of creatine kinase in the supernatant is determined from an assay which couples ATP formation from ADP and creatine phosphate with the formation of DADPH (measured by absorbance at 340 nm), according to Shainberg, *et al*. (34). Briefly, 100 µl of the supernatant are mixed with 890 µl of reaction mixture and the reaction is initiated by adding 10 µl of creatine phosphate (15 mM). The change in adsorbance at 340 nm is measured in a UV160 spectrophotometer (Shimidazu, Tokyo) and the slope of the change in absorbance per min. is used to determine creatine kinase activity.

### Autoradiography

First-passage human MSCs and periosteal cells are dispersed into 100-mm dishes at a density of 4.0 x 10⁵ cells in 5 ml of complete medium. After 24 hours, 2.5 µCi of [methyl-³H]thymidine (Amersham, Arlington Heights, IL; 0.5 µCi/ml) are added to each dish. The cells are incubated for 48 hours, washed twice with Tyrode's salts solution and Complete Medium is added to chase unincorporated radioisotope for 48 hours. Subsequently, cells were harvested by digestion with trypsin and counted in a hemocytometer. The cell density is adjusted to 5.0 x 10⁴ cells/ml for MSCs, and 1.0 x 10⁵ cell/ml for periosteum-derived cells. Aliquots of 1.0 ml of each cell suspension are added to the dishes into which twice the number of myoblasts are plated.

At 2-day intervals up to 11 days, co-culture plates are harvested for autoradiography. Randomly chosen dishes are washed twice with cold Tyrode's salts solution, and the cells are fixed with 10% neutral formalin. After the cells are dehydrated in graded ethanol and air-dried, the dishes are coated with NTB-2 autoradiography emulsion (Eastman Kodak, Rochester, NY) and stored in the dark at -20°C for 7 days. Subsequently, the dishes are developed and air-dried, and the cells are observed under a microscope (BH-2, Olympus, Tokyo).

### Autoradiography with Immunohistochemistry

Co-cultures of MSCs with myoblasts are immunostained with anti-dystrophin antibody prior to autoradiography. At 9 days, the dishes are washed twice with Tyrode's salts solution and the cells are fixed with cold methanol for 15 min. After washing with Tyrode's salts solution, PBS+HS (phosphate buffered saline pH 7.4, containing 10% horse serum; JRH Biosciences, Lenesa, KS) is added onto the cells. The cells are incubated for 2.5 hours in a humid chamber with anti-dystrophin antibody diluted to 1 to 250 with PBS+HS. After incubation, the cells are washed 4 times with PBS+HS for 5 min. each and incubated with biotinylated sheep anti-sheep IgG (Vector Laboratories, Inc., Burlingame, CA) and diluted (1:500) in PBS +HS. After washing 4 times, the cells are exposed to streptavidin-horseradish peroxidase conjugate (Bethesda Research Laboratories, Life Technologies Inc., Gaithersburg, MD) for 30 min. To develop color, the cells are immersed in the solution of diaminobenzidine (DAB) (0.2 mg/ml) in 20 mM (tris)hydroxyethyl aminoethane, 150 mM sodium chloride, pH 7.6 containing hydrogen peroxide (0.01%) at room temperature for 15 min. in the dark. The cells are washed once with PBS and twice with distilled water and then exposed to 10% copper nitrate for 1 min. to intensify the DAB product. After washing again with distilled water, cells are then dehydrated in graded ethanol and air-dried, and autoradiography is performed as described above.

### Cited Literature

1. Anderson, JB, Ovalle WK, Bressler BH: Electron microscopic and autoradiographic characterization of hind limb muscle regeneration in the mdx mouse. Anat. Rec. (1987) 219:243-257.
2. Archer, CW, Langille RM, Teran MA and Solursh M: Myogenic potential of chick limb bud mesenchymal in micromass culture. Anat. Embryol. Berl. 1992;185:299-306.
3. Ashton, BA, Allen, TD, Howlett, CR, Eagleson, CC, Hattori A, Owen M: Formation of bone and catilage by marrow stromal cells in diffusion chambers in vivo. Clin. Orthop. Rel. Res. 1980;151:294-307.
4. Bruder, SP, Gazit, D, Passi-Bven, L, Bab, I, Caplan AI: Osteochondral differentiation and the emergence of stage-specific osteogenic cell-surface molecules by bone marrow cells in diffusion chambers. Bone and Mineral 1990;11:141-151.
5. Bulfied, G, Siller WG, Wight PAL, Moore KJ: X chromosome-linked muscular dystrophy (mdx) in the mouse. Proc. Natl. Acad. Sci. USA 1984;81:1189-1192.
6. Caplan, AI: Simplified procedure for preparing myogenic cells for culture. J. Embryol. Expl. Morph. 1965;36:175-181.
7. Caplan, AI: (1991) Mesenchymal stem cells. J. Orthop. Res. 1991;9:641-650.
8. Caplan, AI: The molecular control of muscle and cartilage development, in Subtelney S, Abbott U (eds.): 39th Annual Symposium of the Society for Developmental Biology, New York, Alan R. Liss, Inc., 1981, pp 37-68.
9. Chaudhari N, Beam KG: Fibroblasts fuse with myotubes developing in culture, in Griggs R, Karpati G (eds): Myoblast Transfer Therapy Plenum Press, New York, 1990. pp 131-137.
10. Coulton, GR, Morgan JE, Partridge TA, Sloper JC: The mdx mouse skeletal muscle myopathy: I. A histological, morphometric and biochemical investigation. Neuropathol. Appl. Neurobiol. 1988;14:53-70.
11. Constantinides, PG, Jones PA, Gevers W: Functional striated muscle cells from nonmyoblast precursors following 5-azacytidine treatment. Nature 1977;267:364-366.
12. Friedenstein, AJ: Determined and incucible osteogenic precursor cells, in Elliot K, Fitzsimons, D (eds): Hard Tissue Growth, Repair and Remineralization, Ciba Found. Symp., I, North-Holland, Amsterdam: Elsevier-Excerpta Medica, 1973. vol 11, pp 169-185.
13. Priedenstein, AJ, Chailkhyan RK, Gerasimov UV: Bone marrow osteogenic stem cells: in vitro cultivation and transplantation in diffusion chambers. Cell Tissue Kinet. 1987;20:263-272.
14. Frair, PM, Peterson AC: The nuclear cytoplasmic relationship in 'Mosaic' skeletal muscle fibers from mouse chimaeras. Exp. Cell Res. 1983;145:167-178.
15. Garcia L, dreyfus P, Pincon-Raymont M, Billageois A, Chassande O, Romay G, Lazdunski M, Reiger P: Phenotypic and functional reversion of muscular dysgenesis by heterotypic fibroblast-myotube fusion in vitro, in Griggs R, Karpati G (eds): Myoblast Transfer Therapy. New York, Plenum Press, 1990. pp 139-146.
16. Goshima, J, Goldberg VM, Caplan AI: The origin of bone formed in composite grafts of porous calcium phosphate ceramic loaded with marrow cells. Clin. Orthop. 1991;269:274-283.
17. Grigoriadis, AE, Heersche JNM, Aubin JE: Differentiation of muscle, fat, cartilage and bone from progenitor cells present in a bone-derived clonal cell population: effect of dexamethasone. J. Cell Biol. 1988;106:2139-2151.
18. Gussoni, E, Paviath, GK, Lanctot AM, Sharma KR, Miller RG, Steinman L, Blau HM: Normal dystrophin transcripts detected in Duchenne muscular dystrophy patients after myoblast transplantation. Nature (1992)356:435-438.
19. Hauschka, SD: Clonal analysis of vertebrate myogenesis III. Developmental changes in the muscle colony-forming cells of the human fetal limb. Dev. Biol. (1974);37:345368.
20. Holtzer H, Bischoff R: Mitosis and myogenesis, in Briskey BJ, Cassens, RG, March BB (eds): The physiology and biochemistry of muscle as a food. Madison Wisconsin, Univ. of Wisconsin Press, Madison, Wisconsin, 1970. pp 29-51.
21. Hustad CM, Jones PA: Effect of myogenic determination on tumorigenicity of chemically transformed 10t1/2 cells. Mol. Carcinogenesis 1991;4:153-161.
22. Hoffman EP, Morgan JE, Watkins SC, Partridge TA: Somatic reversion/suppression of the mouse mdx phenotype in vivo. J. Neurol. Sci. 1990:99;9-25.
23. Karpati G, Pouliot Y, Zubrzycka-Gaam B, Carpenter S, Ray PN, Worton RG, Holland P: Dystrophin is expressed in mdx skeletal muscle fibers after normal myoblast implantation. Am. J. Pathol. 1989;135:28-32.
24. Law PK, Goodwin TG, Wang MG: Normal myoblast injections provide genetic treatment for murine dystrophy. Muscle & Nerve 1988;11:525-533.
25. Morgan JB, Hoffman EP, Partridge TA: Normal myogenic cells from newborn mice restore normal histology to degenerating muscles of the mdx mouse. J. Cell Biol. 1990;111:2437-2447.
26. Morgan JB, Partridge TA: Cell transplantation and gene therapy in muscular dystrophy. Bioassay 1992;14:641-645.
27. Nakahara H, Bruder SP, Haynesworth SE, Holecek JJ, Baber MA, Goldberg VM, Caplan AI: Bone and cartilage formation in diffusion chambers by subcultured cells derived from the periosteum. Bone 1990;11:181-188.
28. Oghushi H, Goldberg VM, Caplan AI: Heterotopic osteogenesis in porous ceramics induced by marrow cells. J. Orthop. Res. 1989;7:568-578.
29. Owen ME, Friedenstein AJ: Stromal stem cells: marrow-derived osteogenic precursors in Bvered D, Harcett S (eds): Cell and Molecular Biology of Vertebrate Hard Tissue, Ciba Foundation Symposium. Chichester, John Wiley and Sons, 1988. vol 136 pp 42-60.
30. Partridge TA, Sloper JC: Evidence of fusion between host and donor myoblasts in skeletal muscle grafts. Nature 1978;273:306-308.
31. Partridge TA, Morgan JB, Coulton GR, Hoffman EP, Kunkel LM: Conversion of mdx myofibers from dystrophin-negative to -positive by injection of normal myoblasts. Nature 1989;337:176-179.
32. Phillips GD, Hoffman JR, Knighton DR: Migration of myogenic cells in the rat extensor digitorum longus muscle studies with a split autograft model. Cell Tissue Res. 1990;262:81-88.
33. Sasse J, Horvitz A, Pacifici M, Holtzer H: Separation of precursor myogenic and chondrogenic cells in early limb bud mesenchyme by a monoclonal antibody. J. Cell Biol. 1984;99:1856-1866.
34. Shainberg A, Yagh G, Yaffe D: Alterations of enzymatic activities during muscle differentiation in vitro. Devel. Biol. 1971;25:1-29.
35. Solursh M, Reiter RS, Ahrens PB, Vertel BM: Stage- and position-related changes in chondrogenic response of chick embryonic wing mesenchyme to treatment with dibutyryl cyclic AMP. Dev. Biol. 1981;83:9-19.
36. Swalla BJ, Solursh M: The independence of myogenesis and chondrogenesis in micromass cultures of chick wing buds. Dev. Biol. 1986;116:31-38.
37. Taylor SM, Jones PA: Multiple new phenotypes induced in 1Ot1/2 and 3T3 cells treated with 5-acacytidine. Cell 1979;17:771-779.
38. Wakelam MJ0: Myoblast fusion-a mechanistic analysis, in: Current Topics in membranes and transport. New York, Academic Press 1988;12:87-107.
39. Watkins SC, Hoffman EP, Slayter HS, Kunkel LM: Dystrophin distribution in heterozygote mdx mice. Muscle & Nerve 1989;12:861-868.
40. Yaffe D, Feldman M: The formation of hybrid multinucleated muscle fibers from myoblasts of different genetic origin. Dev. Biol. 1965;11:300-317.

## Claims

1. A compositions of matter which comprises a mixture of isolated human muscle precursor cells and isolated human mesenchymal stem cells, with the proviso that the stem cells are not embryonic stem cells.

2. The composition of claim 1 wherein the mesenchymal stem cells are marrow-derived.

3. The composition of claim 1 wherein the mesenchymal stem cells are periosteum-derived.

4. The composition of claim 1 which further comprises complete medium.

5. The composition of claim 1 which further comprises a myoinductive agent.

6. The composition of claim 5 wherein the myoinductive agent is selected from the group consisting of 5-azacytidine and 5-azadeoxycytidine.

7. A composition of matter which comprises a mixture of isolated muscle precursor cells and isolated periosteum cells.

8. The composition of claim 7 wherein there are at least as many muscle precursor cells in the mixture as there are periosteum cells.

9. The composition of claim 7 which further comprises a myoinductive agent.

10. The composition of claim 7 wherein the myoinductive agent is selected from the group consisting of 5-azacytidine and 5-azadeoxycytidine.

11. A method for inducing isolated human mesenchymal stem cells to differentiate into myogenic cells which comprises maintaining said cells in the presence of muscle precursor cells,
wherein the isolated human mesenchymal stem cells are maintained in the presence of muscle precursor cells *in vitro* with the proviso that the stem cells are not embryonic stem cells.

12. Use of a myogenic cell producing amount of isolated mesenchymal stem cells or use of a myogenic cell producing amount of the composition of claims 1, 5 or 7 for the preparation of a medicament for producing dystrophin-positive myogenic cells in a mammal.

13. Use of a muscle regenerative amount of isolated mesenchymal stem cells or use of a muscle regenerative amount of the composition of claims 1, 5 or 7 for the preparation of a medicament for effecting muscle regeneration in an individual in need thereof.

14. Use of an amount of isolated mesenchymal stem cells effective to produce dystrophin-positive myogenic cells in an individual or use of an amount of the composition of claims 1, 5 or 7 effective to produce dystrophin-positive myogenic cells in an individual for the preparation of a medicament for treating muscular dystrophy in an individual so afflicted.

## Patentansprüche

1. Zusammensetzung, die ein Gemisch aus isolierten menschlichen Muskel-Vorläuferzellen und isolierten menschlichen mesenchymalen Stammzellen umfasst, mit der Maßgabe, dass die Stammzellen keine embryonalen Stammzellen sind.

2. Zusammensetzung nach Anspruch 1, wobei die mesenchymalen Stammzellen von Knochenmark abgeleitet sind.

3. Zusammensetzung nach Anspruch 1, wobei die mesenchymalen Stammzellen von Periost abgeleitet sind.

4. Zusammensetzung nach Anspruch 1, die ferner Vollmedium umfasst.

5. Zusammensetzung nach Anspruch 1, die ferner ein Muskel-induzierendes Mittel umfasst.

6. Zusammensetzung nach Anspruch 5, wobei das Muskel-induzierende Mittel ausgewählt ist aus der Gruppe, bestehend aus 5-Azacytidin und 5-Azadesoxycytidin.

7. Zusammensetzung, die ein Gemisch aus isolierten Muskel-Vorläuferzellen und isolierten Periostzellen umfasst.

8. Zusammensetzung nach Anspruch 7, wobei in dem Gemisch mindestens so viele Muskel-Vorläuferzellen wie Periostzellen vorliegen.

9. Zusammensetzung nach Anspruch 7, die ferner ein Muskel-induzierendes Mittel umfasst.

10. Zusammensetzung nach Anspruch 7, wobei das Muskel-induzierende Mittel ausgewählt ist aus der Gruppe, bestehend aus 5-Azacytidin und 5-Azadesoxycytidin.

11. Verfahren zum Induzieren von isolierten menschlichen mesenchymalen Stammzellen, in myogene Zellen zu differenzieren, das ein Halten der Zellen in Gegenwart von Muskel-Vorläuferzellen umfasst, wobei die isolierten menschlichen mesenchymalen Stammzellen in Gegenwart von Muskel-Vorläuferzellen in vitro gehalten werden, mit der Maßgabe, dass die Stammzellen keine embryonalen Stammzellen sind.

12. Verwendung einer myogene Zellen-produzierenden Menge von isolierten mesenchymalen Stammzellen oder Verwendung einer myogene Zellen-produzierenden Menge der Zusammensetzung nach Anspruch 1, 5 oder 7 zur Herstellung eines Medikaments für eine Produktion Dystrophin-positiver myogener Zellen in einem Säuger.

13. Verwendung einer Muskel-regenerierenden Menge von isolierten mesenchymalen Stammzellen oder Verwendung einer Muskel-regenerierenden Menge der Zusammensetzung nach Anspruch 1, 5 oder 7 zur Herstellung eines Medikaments für ein Bewirken von Muskelregeneration in einem Individuum, das einen Bedarf dafür aufweist.

14. Verwendung einer Menge an isolierten mesenchymalen Stammzellen, die wirksam ist, Dystrophin-positive myogene Zellen in einem Individuum zu produzieren, oder Verwendung einer Menge der Zusammensetzung nach Anspruch 1, 5 oder 7, die wirksam ist, Dystrophin-positive myogene Zellen in einem Individuum zu produzieren, für die Herstellung eines Medikaments zur Behandlung von Muskeldystrophie in einem daran erkrankten Individuum.

## Revendications

1. Composition de matière qui comprend un mélange de cellules précurseurs de muscle humaines isolées et de cellules souches mésenchymateuses humaines isolées, avec pour condition que les cellules souches ne sont pas des cellules souches embryonnaires.

2. Composition selon la revendication 1, dans laquelle les cellules souches mésenchymateuses sont dérivées de la moelle.

3. Composition selon la revendication 1, dans laquelle les cellules souches mésenchymateuses sont dérivées du périoste.

4. Composition selon la revendication 1, qui comprend en outre un milieu complet.

5. Composition selon la revendication 1, qui comprend en outre un agent myoinducteur.

6. Composition selon la revendication 5, dans laquelle l'agent myoinducteur est choisi dans le groupe consistant en 5-azacytidine et 5-azadésoxycytidine.

7. Composition de matière qui comprend un mélange de cellules précurseurs de muscle isolées et de cellules de périoste isolées.

8. Composition selon la revendication 7, dans laquelle il existe au moins autant de cellules précurseurs de muscle dans le mélange qu'il existe de cellules de périoste.

9. Composition selon la revendication 7, qui comprend en outre un agent myoinducteur.

10. Composition selon la revendication 7, dans laquelle l'agent myoinducteur est choisi dans le groupe consistant en 5-azacytidine et 5-azadésoxycytidine.

11. Procédé pour l'induction de cellules souches mésenchymateuses humaines isolées pour la différentiation en des cellules myogéniques, qui comprend le maintient des dites cellules en présence de cellules précurseurs de muscle, tandis que les cellules souches mésenchymateuses humaines isolées sont maintenues en présence de cellules précurseurs de muscle *in vitro*, avec pour condition que les cellules souches ne sont pas des cellules souches embryonnaires.

12. Utilisation d'une quantité produisant des cellules myogéniques de cellules souches mésenchymateuses isolées ou utilisation d'une quantité produisant des cellules myogéniques de la composition selon les revendications 1, 5 ou 7 pour la préparation d'un médicament pour la production de cellules myogéniques positives à la dystrophine chez un mammifère.

13. Utilisation d'une quantité régénératrice des muscles de cellules souches mésenchymateuses isolées ou utilisation d'une quantité régénératrices des muscles de la composition selon les revendications 1, 5 ou 7 pour la préparation d'un médicament pour effectuer la régénération des muscles chez un individu qui en a besoin.

14. Utilisation d'une quantité de cellules souches mésenchymateuses isolées efficace pour la production de cellules myogéniques positives à la dystrophine chez un individu pour utilisation d'une quantité de la composition selon les revendications 1, 5 ou 7 efficace pour la production de cellules myogéniques positives à la dystrophine chez un individu pour la préparation d'un médicament pour le traitement de la dystrophie musculaire chez un individu qui en souffre.
